# EUROPEAN PATENT SPECIFICATION

(11) **EP 3 263 308 B1**
(45) Date of publication and mention of the grant of the patent: **25.10.2023**
(21) Application number: 16803113.6
(22) Date of filing: 23.05.2016
(51) Int. Cl.: B29C 35/02, B29C 35/08, B29C 37/00, B29C 70/46, B29C 70/02, C08J 5/24, B29K 105/16

(54) **CURING DEVICE AND CURING METHOD FOR RESIN COMPOSITE MATERIAL**
HÄRTUNGSVORRICHTUNG UND HÄRTUNGSVERFAHREN FÜR HARZVERBUNDMATERIAL
DISPOSITIF DE DURCISSEMENT ET PROCÉDÉ DE DURCISSEMENT POUR MATÉRIAU COMPOSITE DE RÉSINE

(30) Priority: 03.06.2015 JP 2015113321
(43) Date of publication of application: 03.01.2018
(73) Proprietor: Mitsubishi Heavy Industries, Ltd., Tokyo 108-8215 (JP)
(72) Inventor: KAMIHARA, Nobuyuki, Tokyo 108-8215 (JP); ABE, Toshio, Tokyo 108-8215 (JP)
(74) Representative: Henkel & Partner mbB
(86) International application number: PCT/JP2016/065170
(87) International publication number: WO 2016/194678

(56) References cited:
- WO-A1-98/00274
- WO-A1-2009/127864
- WO-A1-2015/025166
- JP-A- 2002 219 754
- JP-A- 2013 238 791
- JP-A- 2015 059 123
- US-A- 4 859 524
- US-A- 5 032 327
- US-A1- 2010 136 866
- US-A1- 2012 168 990

## Description

### Technical Field

The present invention relates to a curing device for a resin composite material, and to a curing method for a resin composite material.

### Background Art

A reinforced resin composite material (fiber reinforced plastic) obtained by impregnating a fibrous reinforcing material such as glass fibers or carbon fibers into a matrix resin is a lightweight material having high strength and high elasticity and is widely used in an aerospace industry, an automobile industry, sports equipment, or the like.

As the matrix resin of the resin composite material, a thermosetting resin or a thermoplastic resin is suitably used since the resins have a short curing time and high productivity. In recent years, an intermediate molding material is frequently used, which is referred to as a flexible prepreg which is semi-cured by impregnating a thermosetting or thermoplastic matrix resin to a mat-shaped fibrous reinforcing material.

In general, as described in PTL 1, in a case where a molded resin article is molded by a prepreg, the prepreg is laminated on or wound around a molding tool, a jig, or the like of a manufactured molded resin article to be molded into a predetermined shape, and the prepreg is enclosed by a vacuum bag so as to be evacuated in order to increase adhesion with respect to the molding tool, the jig, or the like. Thereafter, the prepreg is accommodated in a heater such as an autoclave, and the matrix resin of the prepreg is cured while being heated and pressurized. In this way, if pressurization is not applied to the prepreg simultaneously with heating in order to cure the prepreg, a favorable curing reaction cannot be obtained, and predetermined strength cannot be obtained due to bubbles of air included inside the prepreg or bubbles of gas generated when the prepreg is heated remaining.

PTL 2 discloses a tool and a method for curing curable resinous composite materials including fiber-reinforced curable resinous composite materials using microwaves. The tool comprises a tool body having a mold cavity defined at least in part by a mold surface on which a composite material can be molded, a microwave source located in or on the tool body to emit microwaves into the cavity to at least partially cure composite material on the mold surface, and pressurization means arranged to facilitate consolidation of the composite material during cure, wherein the microwave source preferably comprises one or more magnetrons. In order to increase the heating effect of the microwaves on the composite material, the composite material may include a conductive material comprising metallic particles such as steel powder or aluminum powder.

PTL 3 discloses an apparatus and a method for press-molding a resin material at good working efficiency and reduced energy consumption. The apparatus comprises a press, a pair of metal molds respectively arranged in the press so as to be insulated from a ground with insulating materials, a molding portion including the pair of metal molds, electrodes provided at two points on either side of the molding portion so as to flank the molding portion, and a high frequency current generator electrically connected to the electrodes flanking the molding portion and applying high frequency current having a frequency of about 10 kHz or more between the electrodes. The resin material to be press-molded may be a fiber-reinforced composite material such as a prepreg having a fabric of reinforcement fibers impregnated with a thermoplastic resin.

PTL 4 discloses an apparatus and a method for curing composite materials including fiber-reinforced curable resinous composite materials in short time without using an autoclave or a heat blanket. The apparatus comprises a mold for shaping an article having a matrix of fibers and resin, and means for curing the article by magnetic induction heating or/and resistive heating, wherein the means for curing the article by magnetic induction heating or/and resistive heating may be magnetic induction heating coils. In order to improve the heating effect of the magnetic induction heating coils on the article to be cured, the article may include an electrically conductive constituent, wherein the electrically conductive constituent preferably comprises carbon fibers.

### Citation List

### Patent Literature

[PTL 1] JP 2011-152753 A
[PTL 2] WO 2015/025166 A1
[PTL 3] US 2012/168990 A1
[PTL 4] WO 98/00274 A1

### Summary of Invention

### Technical Problem

Some layers of sheet-shaped intermediate molding materials such as prepregs are laminated, are accommodated in a vacuum bag so as to be evacuated and are heated. Thus, the intermediate molding materials are molded as a molded resin article.

When the intermediate molding material is heated, it is not possible to increase the temperature of the intermediate molding material if not only the intermediate molding material but also all portions including a molding tool, a jig, or the like are not heated by the heater. Accordingly, a heating process takes a long period of time, and energy consumed for the heating is excessive.

For these reasons, a molded resin article which is manufactured by laminating the intermediate molding materials is inevitably expensive due to high manufacturing costs thereof.

The present invention is made in consideration of the above-described circumstance, and an object thereof is to provide a curing device for a resin composite material, and a curing method for a resin composite material capable of saving labor when an intermediate molding material is molded, simplifying curing equipment, saving energy, and decreasing manufacturing costs of a molded resin article. The present invention is defined by the claims.

### Solution to Problem

In a first aspect, the present invention thus relates to a curing device for a resin composite material as defined in any one of claims 1, 2 and 3. The curing devices include:
an environment setting unit which is adapted for applying a specific physical environment to an uncured resin composite material including a fibrous reinforcing material, a thermosetting or thermoplastic matrix resin, and a metal nanomaterial selected from platinum, gold, nickel and copper, wherein the specific physical environment is such that the molecular momentum of the metal nanomaterial is increased and the metal nanomaterial self-heats when placed in the specific physical environment;
a pressurizing body which is adapted for coming into pressure-contact with a surface of the resin composite material; and
a pressure-contact driving unit which allows the pressurizing body to come into pressure-contact with the surface of the resin composite material.

According to the curing devices having the above-described configuration, if the specific physical environment in which the molecular momentum of the object increases is applied to the uncured resin composite material by the environment setting unit, the molecular momentum of the metal nanomaterial included in the resin composite material increases, the metal nanomaterial is self-heated, the temperature of the resin composite material increases, and the resin composite material is softened.

In this state, the pressurizing body comes into pressure-contact with the surface of the resin composite material by the pressure-contact driving unit, the temperature of the resin composite material increases, and the softened resin composite material is pressurized by the pressurizing body. In this case, bubbles of air included in the resin composite material or bubbles of gas generated during heating are removed, and the resin composite material is molded into the shape of a molded resin article.

In a case where the matrix resin of the resin composite material is a thermosetting resin, a curing reaction is generated by the pressurization, and the resin composite material is cured by the curing reaction. Meanwhile, in a case where the matrix resin of the resin composite material is a thermoplastic resin, the resin composite material is cured by cooling the thermoplastic resin.

The pressurizing body may not be set to a size which comes into pressure-contact with the entire surface of the molded resin article manufactured by the resin composite material. That is, the pressurizing body is formed to have a size or a shape which comes into pressure-contact with only a portion of the surface of the resin composite material (molded resin article), and the entire resin composite material can be pressurized and cured by repeating pressurization with respect to the surface of the resin composite material partially by using the pressurizing body.

Moreover, since the resin composite material is heated by self-heating properties of the metal nanomaterial and the heated resin composite material is pressurized by the pressurizing body, unlike the related art, the resin composite material is not required to be enclosed and evacuated by a vacuum bag along with a molding tool, a jig, or the like so as to be pressurized.

Accordingly, unlike the related art, it is not necessary to prepare a vacuum bag capable of accommodating the entire resin composite material (molded resin article) or a heater such as an autoclave having a large capacity, and it is possible to greatly simplify the curing equipment.

In addition, since only the resin composite material is heated by applying the specific physical environment in which the molecular momentum increases by the environment setting unit, unlike the case where the heater of the related art is used, energy for heating all portions including the molding tool, the jig, or the like is not required, and it is possible to save energy.

Accordingly, it is possible to remarkably decrease the manufacturing costs of the molded resin article.

In the curing device of claim 1, the environment setting unit is an electromagnetic wave irradiation unit, and the specific physical environment is a state where electromagnetic waves are emitted by the electromagnetic wave irradiation unit.

In this way, by irradiating the uncured resin composite material into which the metal nanomaterial is included with the electromagnetic waves, the molecular momentum of the metal nanomaterial increases, the metal nanomaterial is rapidly self-heated, and thus, it is possible to heat the resin composite material.

In the curing device of claim 2, the environment setting unit is a plurality of electrodes which directly face each other in a state where the resin composite material is interposed between the electrodes, and the specific physical environment is a state where an electric field is applied to portions between the plurality of electrodes.

In this way, by applying the electric field to portions between a plurality of electrodes which directly face each other in a state where the resin composite material is interposed between the electrodes, similarly to the case where the electromagnetic waves are emitted, it is possible to heat the resin composite material by increasing the molecular momentum of the metal nanomaterial. Since the configuration of applying the electric field is simpler than the configuration of emitting the electromagnetic waves, it is possible to further simplify the curing equipment, which can contribute to the decrease in the manufacturing costs of the molded resin article.

In the curing device of claim 3, the environment setting unit is a magnetic field applying coil, and the specific physical environment is a state where a magnetic field is applied to the resin composite material by the magnetic field applying coil.

In this case, compared to the cases where the electromagnetic waves are emitted or the electric fields are applied, effects of self-heating the metal nanomaterial included in the resin composite material are inferior. However, the configuration of the curing equipment can become simpler and cheaper. Accordingly, it is possible to effectively heat the resin composite material according to properties of the used metal nanomaterial.

In the curing devices of the present invention, a material of the pressurizing body may be any one of quartz glass, a polymer material, and a ceramics material.

Since the quartz glass, the polymer material, or the ceramics material is easily self-heated even when placed in a physical environment such as the electromagnetic waves, the electric field, or the magnetic field, the temperatures of these do not increase unlike the resin composite material, and a function as the pressurizing body is not damaged.

In the curing devices of the present invention, a plurality of pressurizing bodies may be continuously provided in a predetermined direction of the resin composite material and a pressurizing body positioned at an arbitrary position among the pressurizing bodies can be operated.

Accordingly, for example, one pressurizing body comes into pressure-contact with one location (for example, center portion) of a sheet-shaped resin composite material and other pressurizing bodies adjacent to the one pressurizing body sequentially come into pressure-contact with the resin composite material. Accordingly, a curing range of the resin composite material is widened, bubbles of air or gas included in the resin composite material are extracted to the end portion sides of the resin composite material, and favorable degassing can be achieved.

In the curing devices of the present invention, a plurality of environment setting units may be continuously provided in a predetermined direction of the resin composite material and an environment setting unit positioned at an arbitrary position among the environment setting units can be operated.

Accordingly, by applying the specific physical environment in which the molecular momentum of the object is increased by the environment setting unit to an arbitrary portion of the resin composite material, the metal nanomaterial of this portion is self-heated so as to be heated, and thus, it is possible to cure the resin composite material. In addition, by sequentially shifting the locations to be heated, the curing range of the resin composite material is widened, bubbles of air or gas included in the resin composite material are extracted to the end portion sides of the resin composite material, and thus, favorable degassing can be achieved.

In a second aspect, the present invention relates to a curing method for a resin composite material as defined in any one of claims 7, 8 and 9. The curing methods include:
a heating step of applying a specific physical environment to an uncured resin composite material including a fibrous reinforcing material, a thermosetting or thermoplastic matrix resin, and a metal nanomaterial selected from platinum, gold, nickel and copper, wherein the specific physical environment is such that the molecular momentum of the metal nanomaterial is increased and the metal nanomaterial self-heats, and heating the resin composite material; and
a pressurization step of allowing a pressurizing body to come into pressure-contact with the surface of the heated resin composite material and pressurizing the heated resin composite material.

According to the curing methods, first, in the heating step, the specific physical environment in which the metal nanomaterial is self-heated is applied to the uncured resin composite material. Accordingly, the molecular momentum of the metal nanomaterial included in the resin composite material increases, the metal nanomaterial is self-heated, the temperature of the resin composite material increases, and thus, the resin composite material is softened.

Next, in the pressurization step, the pressurizing body comes into pressure-contact with the surface of the heated resin composite material, the temperature of the resin composite material increases, and the softened resin composite material is pressurized. In this case, bubbles of air included in the resin composite material or bubbles of gas generated during heating are removed, and the resin composite material can be molded into the shape of a molded resin article.

In a case where the matrix resin of the resin composite material is a thermosetting resin, a curing reaction is generated by the pressurization, and the resin composite material is cured by the curing reaction. Meanwhile, in a case where the matrix resin of the resin composite material is a thermoplastic resin, the resin composite material is cured by cooling the thermoplastic resin.

The pressurizing body may not be set to a size which comes into pressure-contact with the entire surface of the molded resin article manufactured by the resin composite material, and it is possible to cure the entire resin composite material by repeatedly pressurizing the surface of the resin composite material using the pressurizing body.

Moreover, since the resin composite material is heated by self-heating properties of the metal nanomaterial and the heated resin composite material is pressurized by the pressurizing body, unlike the related art, the resin composite material is not required to be enclosed and evacuated by a vacuum bag along with a molding tool, a jig, or the like so as to be pressurized. Accordingly, unlike the related art, it is not necessary to prepare a vacuum bag capable of accommodating the entire resin composite material (molded resin article) or a heater such as an autoclave having a large capacity, and it is possible to greatly simplify the curing equipment.

In addition, since only the resin composite material is heated by applying the specific physical environment in which the molecular momentum increases, unlike the case where the heater of the related art is used, energy for heating all portions including the molding tool, the jig, or the like is not required, and it is possible to save energy.

Accordingly, it is possible to remarkably decrease the manufacturing costs of the molded resin article.

In the curing method of claim 7, the specific physical environment is a state where electromagnetic waves are emitted.

In this way, by irradiating the uncured resin composite material into which the metal nanomaterial is included with the electromagnetic waves, the molecular momentum of the metal nanomaterial increases, the metal nanomaterial is rapidly self-heated, and thus, it is possible to heat the resin composite material.

In the curing method of claim 8, the specific physical environment is a state where an electric field is applied.

In this way, by applying the electric field to the uncured resin composite material into which the metal nanomaterial is included, similarly to the case where the electromagnetic waves are emitted, the molecular momentum of the metal nanomaterial increases, and thus, it is possible to heat the resin composite material.

In the curing method of claim 9, the specific physical environment is a state where a magnetic field is applied.

In this way, by applying the magnetic field to the uncured resin composite material into which the metal nanomaterial is included, similarly to the cases where the electromagnetic waves are emitted or the electric field is applied, the molecular momentum of the metal nanomaterial increases, and thus, it is possible to heat the resin composite material.

In the curing methods of the present invention, the heating step and the pressurization step may be alternately performed a plurality of times. Accordingly, for example, in a case where some sheet-shaped resin composite materials are laminated, the resin composite material is reliably pressurized and cured every time one resin composite material is laminated, and it is possible to prevent the quality of the shape or the strength of the molded resin article from decreasing.

### Advantageous Effects of Invention

As described above, according to the curing devices for a resin composite material and the curing methods of the present invention, it is possible to save labor when the resin composite material is molded, simplify the curing equipment, save energy, and decrease the manufacturing costs of a molded resin article. In addition, the manufacturing costs of the molded resin article are inexpensive.

### Brief Description of Drawings

Fig. 1 is a plan view showing a curing device for a resin composite material according to a first embodiment of the present invention.
Fig. 2 is a longitudinal sectional view of the curing device taken along line II-II of Fig. 1.
Fig. 3 is a flowchart showing a curing method according to the present invention.
Fig. 4 is a plan view showing a curing device for a resin composite material according to a second embodiment of the present invention.
Fig. 5 is a plan view showing a curing device for a resin composite material according to a third embodiment of the present invention.
Fig. 6 is a plan view showing a curing device for a resin composite material according to a fourth embodiment of the present invention.

### Description of Embodiments

Hereinafter, embodiments of the present invention will be described with reference to the drawings.

### [First Embodiment]

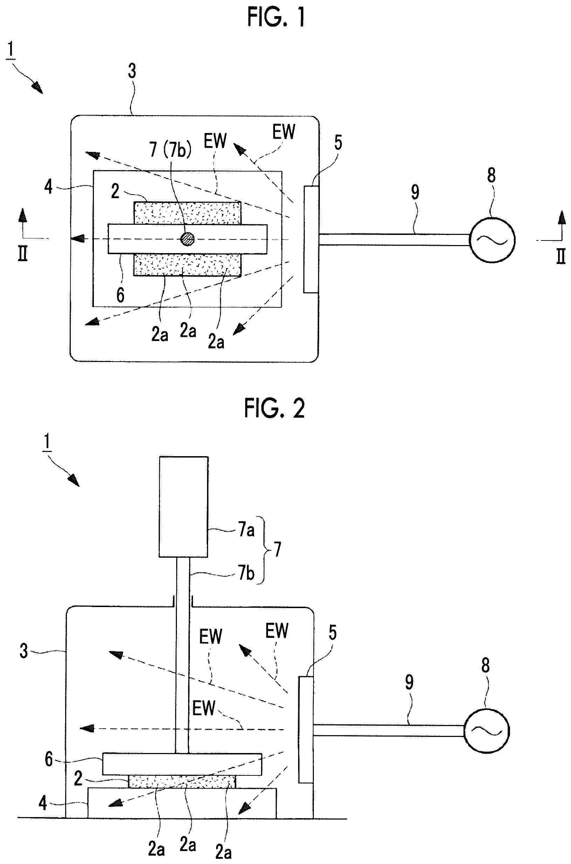

Fig. 1 a plan view showing a curing device according to a first embodiment of the present invention, and Fig. 2 is a longitudinal sectional view of the curing device taken along line II-II of Fig. 1.

For example, the curing device 1 is a device which heats and cures an uncured prepreg 2 (resin composite material) while pressurizing the prepreg 2 in a thickness direction thereof. As well known, the prepreg 2 is an intermediate molding material obtained by laminating or impregnating a thermosetting or thermoplastic matrix resin on a fibrous reinforcing material such as carbon fibers or glass fibers, and the intermediate molding material is semi-integrated and deformable at room temperature.

For example, the curing device 1 is configured to include a casing-shaped cavity 3, a flat forming base 4 which configures a bottom portion of the cavity 3, an electromagnetic wave irradiation unit 5 (environment setting unit) which is provided on a side inner surface of the cavity 3, a pressurizing body 6, an actuator 7 (pressure-contact driving unit), a high frequency power source 8, and a wave guide pipe 9. The prepreg 2 is laminated on the forming base 4 or a molding tool (not shown), a jig (not shown), or the like installed on the forming base 4, and is pressurized from above by the pressurizing body 6 described later. It is considered that the cavity 3 is not provided so as to form the curing device 1 in an open shape.

The prepreg 2 used in the present embodiment is configured such that metal nanomaterials 2a which self-heat when placed in a specific physical environment in which molecular momentum of an object increases, is added or attached to the matrix resin. The metal nanomaterials 2a are metal materials in which a two-dimensional size or a three-dimensional size is nanoscale (one to several hundred nm), and as specific shapes, there are nanofibers, nanocoils, nanoparticles, nanotubes, or the like. Particularly, since the nanocoils or the nanofibers have high electromagnetic wave absorption efficiency, the nanocoils or the nanofibers are preferable.

The material of each of the metal nanomaterials 2a is a metal which has high electromagnetic wave absorption efficiency in frequencies of electromagnetic waves EW emitted from the electromagnetic wave irradiation unit 5. Specifically, the material of the metal nanomaterial 2a is platinum (Pt), gold (Au), nickel (Ni) or copper (Cu). Since the metal nanomaterials 2a absorb the electromagnetic waves EW, the metal nanomaterials 2a increase molecular momentum and self-heat, and thereby, the temperatures of the metal nanomaterials 2a abruptly increase.

If an addition amount of the metal nanomaterials 2a with respect to the matrix resin of the prepreg 2 is excessive, there is a concern that cracks may occur when the prepreg 2 is used as a molded resin article. Accordingly, the addition amount of the metal nanomaterials 2a is 50 pg / cm2 or less, preferably 10 pg / cm2 or less, and more preferably 2 µg / cm2 or less in terms of a weight ratio per unit area. Since the above-described metal nanomaterials 2a have high electromagnetic wave absorption efficiency, even when the addition amount thereof is small, it is possible to obtain a large heating value.

In the curing device 1, a physical environment is formed inside the cavity 3, in which the molecular momentum of the metal nanomaterials 2a included in the matrix resin of the prepreg 2 is increased by emitting the electromagnetic waves EW from the electromagnetic wave irradiation unit 5. The electromagnetic wave irradiation unit 5 emits the electromagnetic waves EW supplied via the wave guide pipe 9 from the high frequency power source 8 into the cavity 3.

The frequencies of the electromagnetic waves EW are not particularly limited. However, for example, preferably, the electromagnetic waves EW are not electromagnetic waves which require special management like X-rays. In addition, preferably, the electromagnetic waves are electromagnetic waves having frequencies in which frequency absorption efficiency with respect to a metal configuring the metal nanomaterial 2a is high. Considering the above, preferably, short waves (HF, 3 MHz to 30 MHz), ultrashort waves (VHF, 30 MHz to 300 MHz), and microwaves (300 MHz to 3 GHz) are emitted. Specifically, electromagnetic waves of an ISM band can be used.

Meanwhile, the pressurizing body 6 is provided to be able to come into pressure-contact with and to be separated from the surface of the prepreg 2, and the material of the pressurizing body 6 requires a material which is not easily self-heated, that is, is not heated to a temperature at which a function as the pressurizing body is damaged even when placed in an environment into which the electromagnetic waves EW are emitted. Specifically, the pressurizing body 6 is formed of quartz glass, a polymer material such as PEEK or polyimide, a ceramics material such as SiC, or the like.

For example, the actuator 7 includes a cylinder 7a which is installed above a top plate of the cavity 3 and a rod 7b which extends to be extendable and contractible from the cylinder 7a and penetrates the top plate of the cavity 3, and the tip of the rod 7b is connected to the pressurizing body 6. However, the present invention is not limited to this configuration, and other configurations may be adopted as long as the pressurizing body 6 can come into pressure-contact with the surface of the prepreg 2. The actuator 7 causes the pressurizing body 6 to come into pressure-contact with the surface of the prepreg 2 in a state where the prepreg 2 is irradiated with the electromagnetic waves EW by the electromagnetic wave irradiation unit 5.

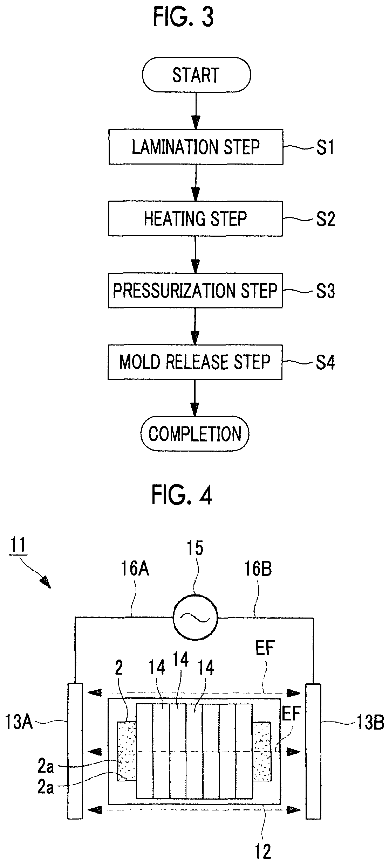

A curing method for the prepreg 2 is performed as follows by the curing device 1 configured as above. In addition, Fig. 3 is a flowchart showing the curing method for the prepreg 2.

First, the uncured prepreg 2 is laminated (placed, wound, or the like) on the forming base 4, a predetermined molding tool, a jig, or the like (lamination step S1).

Next, the electromagnetic wave irradiation unit 5 irradiates the prepreg 2 with the electromagnetic waves EW (heating step S2). The frequencies of the electromagnetic waves EW are not particularly limited. However, for example, preferably, the electromagnetic waves EW are not electromagnetic waves which require special management like X-rays. In addition, preferably, the electromagnetic waves EW are electromagnetic waves having frequencies at which frequency absorption efficiency of the metal configuring the metal nanomaterial 2a is high. Considering the above, preferably, short waves (HF, 3 MHz to 30 MHz), ultrashort waves (VHF, 30 MHz to 300 MHz), and microwaves (300 MHz to 3 GHz) are emitted. Specifically, electromagnetic waves of an ISM band can be used.

If the prepreg 2 is irradiated with electromagnetic waves EW, the metal nanomaterials 2a added to the matrix resin of the prepreg 2 absorb the electromagnetic waves EW. Accordingly, the molecular momentum of the metal nanomaterials 2a increases, the metal nanomaterials 2a self-heat, and the temperatures of the metal nanomaterials 2a abruptly increase. This heat is transmitted to the matrix resin of the prepreg 2, and the matrix resin is softened and melted. The prepreg 2 may be locally irradiated with the electromagnetic waves EW.

Meanwhile, since the pressurizing body 6 is formed of a material which is not easily self-heated even when it is irradiated with the electromagnetic waves EW, the temperature of the pressurizing body 6 is not increased unlike the prepreg 2. Accordingly, a function as the pressurizing body is not damaged.

Next, the pressurizing body 6 comes into pressure-contact with the surface of the uncured prepreg 2 by driving the actuator 7 to pressurize the surface (pressurization step S3). In this case, a pressurizing force is set such that bubbles included inside the prepreg 2 are extracted to the outside.

Bubbles of air included inside the prepreg 2 or bubbles of gas generated during heating are extracted from the center portion of the prepreg 2 toward the peripheral portion thereof by the pressurization of the pressurizing body 6 so as to be removed.

In a case where the matrix resin of the prepreg 2 is a thermosetting resin, a curing reaction is generated by an increase in the temperature due to the self-heating of the metal nanomaterials 2a and the pressurization of the pressurizing body 6, and the matrix resin is cured by the curing reaction. Meanwhile, in a case where the matrix resin of the prepreg 2 is a thermoplastic resin, the matrix resin is cooled after being heated and is cured. If the pressurization is completed, the actuator 7 is driven to lift the pressurizing body 6.

In a case where a range which is pressurized by the pressurizing body 6 is only a portion of the prepreg 2, the pressurization steps S3 are repeated a plurality of times while relative positions between the prepreg 2 and the pressurizing body 6 are shifted. In this case, first, the vicinity of the center portion of the prepreg 2 is pressurized, and the prepreg 2 may be sequentially pressurized to be shifted toward the outside.

In this way, by alternately performing the heating (heating step S2) by the irradiation of the electromagnetic waves EW and the pressurization (pressurization step S3) by the pressurizing body 6 a plurality of times, the entire prepreg 2 is cured, and the molded resin article is formed. The irradiation of the electromagnetic waves EW and the pressurization of the pressurizing body 6 may be simultaneously performed.

Finally, the molded resin article is separated from the forming base 4 and the molded resin article is completed (mold release step S4).

According to the curing device 1 having the above-described configuration, the pressurizing body 6 may not be set to a size which comes into pressure-contact with the entire surface of the molded resin article manufactured by the prepreg 2. That is, the pressurizing body 6 is formed to have a size or a shape which comes into pressure-contact with only a portion of the surface of the prepreg 2 (molded resin article), and the entire prepreg 2 can be pressurized and cured by partially repeating pressurization with respect to the surface of the prepreg 2 using the pressurizing body 6.

Moreover, since the prepreg 2 is heated by self-heating properties of the metal nanomaterials 2a and the heated prepreg 2 is pressurized by the pressurizing body 6, unlike the related art, the prepreg 2 is not required to be enclosed and evacuated by a vacuum bag along with a molding tool, a jig, or the like so as to be pressurized.

Accordingly, unlike the related art, it is not necessary to prepare a vacuum bag capable of accommodating the entire prepreg 2 (molded resin article) or a heater such as an autoclave having a large capacity, and it is possible to greatly simplify the curing equipment.

In addition, since only the prepreg 2 is heated by the emitting of the electromagnetic waves EW from the electromagnetic wave irradiation unit 5, unlike the case where the heater of the related art is used, energy for heating all portions including the molding tool, the jig, or the like is not required. Accordingly, it is possible to greatly save energy, and thus, it is possible to remarkably decrease the manufacturing costs of the molded resin article.

For example, in a case where some sheet-shaped prepregs 2 are laminated, by alternately performing the heating step S2 and the pressurization step S3 every time one prepreg 2 is laminated, the layer of each prepreg 2 is reliably pressurized and cured, and it is possible to prevent the quality of the shape or the strength of the molded resin article from decreasing.

Since it is possible to locally heat the prepreg 2 by limiting the irradiation ranges of the electromagnetic waves EW, by locally heating the molded resin article after curing the entire molded resin article, an addition can be provided or additional molding or additional processing can be performed. The prepreg 2 which is formed in a sheet shape can be laminated by a known lamination method, that is, a lamination method which is manually performed or a lamination method which is performed by an automatic laminating machine.

### [Second Embodiment]

Fig. 4 is a plan view showing a curing device according to a second embodiment of the present invention.

Similarly to the curing device 1 of the first embodiment, a curing device 11 is a device which heats and cures the uncured prepreg 2 (resin composite material) to which metal nanomaterials 2a are added while pressurizing the prepreg 2 in a thickness direction thereof.

The curing device 11 is configured to include a flat forming base 12, a pair of electrodes 13A and 13B (environment setting units) which directly face each other in a state where the prepreg 2 placed on the forming base 12 is interposed therebetween, a plurality of pressurizing bodies 14, a high frequency power source 15, and coaxial cables 16A and 16B.

In the curing device 11, a physical environment is formed, in which molecular momentum of the metal nanomaterials 2a included in the matrix resin of the prepreg 2 is increased by applying electric fields EF between the electrodes 13A and 13B. The electrodes 13A and 13B form the electric fields EF by high frequency current supplied from the high frequency power source 15 via the coaxial cables 16A and 16B.

The plurality of pressurizing bodies 14 are continuously provided in a predetermined direction (for example, a longitudinal direction) of the prepreg 2. The material of each of the pressurizing bodies 14 requires a material which is not easily self-heated, that is, is not heated to a temperature at which a function as the pressurizing body is damaged even when placed in an environment into which the electric fields EF are applied. Each of the plurality of pressurizing bodies 14 can come into pressure-contact with and can be separated from the surface of the prepreg 2 individually by an actuator (not shown). As the actuators, a plurality of actuators similar to the actuator 7 in the curing device 1 of the first embodiment may be disposed. However, other configurations may be adopted.

A curing method for the prepreg 2 is performed as follows by the curing device 11 configured as above. This curing method is also performed by a procedure according to the flowchart shown in Fig. 3.

First, the uncured prepreg 2 is laminated (placed, wound, or the like) on the forming base 12, a predetermined molding tool, a jig, or the like (lamination step S1).

Next, the electrodes 13A and 13B apply the electric fields EF to the prepreg 2 (heating step S2). If the electric fields EF are applied to the prepreg 2, the molecular momentum of the metal nanomaterials 2a added to the matrix resin of the prepreg 2 increases, and the metal nanomaterials 2a are self-heated. This heat is transmitted to the matrix resin of the prepreg 2, and the matrix resin is softened or melted.

Meanwhile, since each of the pressurizing bodies 14 is formed of a material which is not easily self-heated even when it is applied by the electric fields EF, the temperature of the pressurizing body 14 is not increased unlike the prepreg 2. Accordingly, a function as the pressurizing body is not damaged.

Next, the pressurizing bodies 14 come into pressure-contact with the surface of the uncured prepreg 2 by driving the actuators (not shown) to pressurize the surface (pressurization step S3). In this case, a pressurizing force is set such that bubbles included inside the prepreg 2 are extracted to the outside.

Bubbles of air included inside the prepreg 2 or bubbles of gas generated during heating are extracted from the center portion of the prepreg 2 toward the peripheral portion thereof by the pressurization of the pressurizing body 4 so as to be removed.

In a case where the matrix resin of the prepreg 2 is a thermosetting resin, a curing reaction is generated by an increase in the temperature due to the self-heating of the metal nanomaterials 2a and the pressurization of the pressurizing body 6, and the matrix resin is cured by the curing reaction. Meanwhile, in a case where the matrix resin of the prepreg 2 is a thermoplastic resin, the matrix resin is cooled after being heated and is cured. If the pressurization is completed, the actuators are driven to lift the pressurizing bodies 14.

For example, in a case where the prepreg 2 is a sheet shape or an elongated shape, one pressurizing body 14 comes into pressure-contact with one location (for example, center portion) of the prepreg 2, and other pressurizing bodies 14 adjacent to the one pressurizing body 14 sequentially come into pressure-contact with the prepreg 2. Accordingly, a curing range of the prepreg 2 is widened, bubbles of air or gas included in the prepreg 2 are extracted to the end portion sides of the prepreg 2, and favorable degassing can be achieved.

In this way, by alternately performing the heating (heating step S2) by the applying of the electric fields EF and the pressurization (pressurization step S3) by the pressurizing bodies 14 a plurality of times and by sequentially pressurizing the plurality of pressurizing bodies 14 from the center portion toward the outside in the pressurization step S3, it is possible to form the molded resin article such that the entire prepreg 2 is uniformly cured. The applying of the electric fields EF and the pressurization of the pressurizing bodies 14 may be simultaneously performed.

Finally, the molded resin article is separated from the forming base 12 and the molded resin article is completed (mold release step S4).

According to the curing device 11 having the above-described configuration, effects similar to those of the curing device 1 and the curing method of the first embodiment are obtained. In addition to the effects, in the curing device 11, by applying the electric fields EF between the pair of electrodes 13A and 13B directly facing each other in a state where the prepreg 2 is interposed therebetween, similarly to the case of the first embodiment in which the electromagnetic waves are emitted, the molecular momentum of the metal nanomaterials 2a increases, the temperatures of the metal nanomaterials 2a increase, and thus, it is possible to heat the prepreg 2.

Since the high frequency current is supplied from the high frequency power source 15 to the electrodes 13A and 13B via the coaxial cables 16A and 16B, the applying of the electric fields EF can be realized relatively simply. Accordingly, it is possible to realize curing equipment in which a configuration which emits the electromagnetic waves is simple. Accordingly, it is possible to further decrease the manufacturing costs of the molded resin article.

In addition, since the plurality of pressurizing bodies 14 are continuously provided in the predetermined direction of the prepreg 2 and a pressurizing body 14 positioned at an arbitrary position among the plurality of pressurizing bodies 14 can be operated, it is possible to reliably remove bubbles in the prepreg 2 by sequentially performing the pressure-contacts of the pressurizing bodies 14 from one location of the prepreg 2 toward the peripheral portion thereof.

### [Third Embodiment]

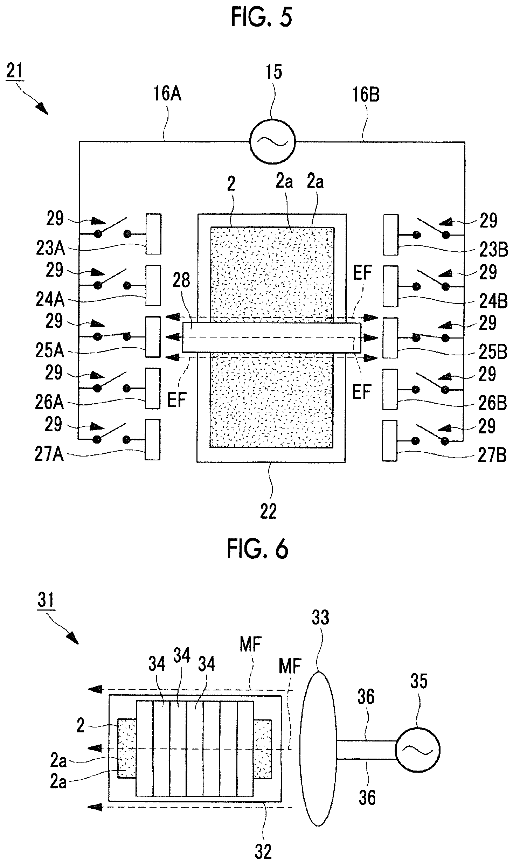

Fig. 5 is a plan view showing a curing device according to a third embodiment of the present invention.

Similarly to the curing device 11 of the second embodiment, a curing device 21 is a device which pressurizes the uncured prepreg 2 (resin composite material) to which the metal nanomaterials 2a in the thickness direction so as to cure the prepreg 2 are added while applying the electric fields EF to the prepreg 2 so as to heat the prepreg 2.

The curing device 21 is configured to include a flat forming base 22, for example, five pairs of electrodes 23A, 23B to 27A, and 27B (environment setting units) which directly face each other in a state where the prepreg 2 placed on the forming base 22 is interposed therebetween, a pressurizing body 28, the high frequency power source 15, and coaxial cables 16A and 16B, and a plurality of switches 29.

The five pairs of electrodes 23A, 23B to 27A, and 27B are continuously provided in a predetermined direction (for example, a longitudinal direction) of the prepreg 2, and are connected to the coaxial cables 16A and 16B via the switches 29. In addition, the electric field EF is applied only between the pair of electrodes in which mutual switches 29 are closed. Accordingly, it is possible to operate an arbitrary pair of electrodes. For example, in Fig. 5, the electric fields EF are applied to between electrodes 25A and 25B positioned at the center.

The pressurizing body 28 is provided to be able to come into pressure-contact with the surface of the prepreg 2, and the material of the pressurizing body 29 is formed of a material which is not easily self-heated even when placed in an environment into which electric fields EF are applied. In the third embodiment, single pressurizing body 28 is provided. However, like the second embodiment, a plurality of pressurizing bodies 29 may be continuously provided in a predetermined direction of the prepreg 2. The pressurizing body 28 can come into pressure-contact with the surface of the prepreg 2 by an actuator (not shown). As the actuator, an actuator similar to the actuator 7 in the curing device 1 of the first embodiment may be used. However, other configurations may be adopted.

A curing method for the prepreg 2 is performed as follows by the curing device 21 configured as above. This curing method is also performed by a procedure according to the flowchart shown in Fig. 3.

First, the uncured prepreg 2 is laminated (placed, wound, or the like) on the forming base 22, a predetermined molding tool, a jig, or the like (lamination step S1).

Next, the electric fields EF are applied to the prepreg 2 by closing the switches 29 of any one pair of electrodes among the five pairs of electrodes 23A, 23B to 27A, and 27B (heating step S2). If the electrode fields EF are applied to the prepreg 2, the molecular momentum of the metal nanomaterials 2a added to the matrix resin of the prepreg 2 increases, and the metal nanomaterials 2a are self-heated. This heat is transmitted to the matrix resin of the prepreg 2, and the matrix resin is softened or melted.

Next, the pressurizing body 28 comes into pressure-contact with the surface of the uncured prepreg 2 by driving the actuators (not shown) to pressurize the surface (pressurization step S3). In this case, the pressurizing body 28 moves to a portion in which the temperature of the matrix resin increases by the applying of the electric fields EF so as to pressurize the portion. That is, among the five pairs of electrodes 23A, 23B to 27A, and 27B, the portion corresponding to the electrode to which the electric fields EF are applied is pressurized by the pressurizing body 28. In a case where the plurality of pressurizing bodies 28 are provided, the pressurizing body 28 corresponding to the portion in which the temperature of the matrix resin increases is pressurized. In this case, a pressurizing force is set such that bubbles included inside the prepreg 2 are extracted to the outside.

Bubbles of air included inside the prepreg 2 or bubbles of gas generated during heating are extracted from the center portion (the positions of the electrodes 25A and 25B) of the prepreg 2 toward the peripheral portion thereof by the pressurization of the pressurizing body 28 so as to be removed.

In a case where the matrix resin of the prepreg 2 is a thermosetting resin, a curing reaction is generated by an increase in the temperature due to the self-heating of the metal nanomaterials 2a and the pressurization of the pressurizing body 6, and the matrix resin is cured by the curing reaction. Meanwhile, in a case where the matrix resin of the prepreg 2 is a thermoplastic resin, the matrix resin is cooled after being heated and is cured. If the pressurization is completed, the actuators are driven to lift the pressurizing body 28.

Finally, the molded resin article is separated from the forming base 22 and the molded resin article is completed (mold release step S4).

According to the curing device 21 having the above-described configuration, effects similar to those of the curing device 11 and the curing method of the second embodiment are obtained. In addition to the effects, in the curing device 21, since the plurality of electrodes 23A, 23B to 27A, and 27B to which the electric fields EF are applied are continuously provided in the predetermined direction of the prepreg 2 and the electrode positioned at an arbitrary position among the plurality of electrodes can be operated, by sequentially operating the electrodes 23A, 23B to 27A, and 27B from one location of the prepreg 2 toward the peripheral thereof, bubbles of air or gas included in the prepreg 2 are extracted to the end portion sides of the prepreg 2, and favorable degassing can be achieved.

### [Fourth Embodiment]

Fig. 6 is a plan view showing a curing device according to a fourth embodiment of the present invention.

A curing device 31 is configured to include a flat forming base 32, a magnetic field applying coil 33 which is installed on one side of the forming base 32, a plurality of pressurizing bodies 34, a power source 35, and power lines 36.

In the curing device 31, a physical environment is formed, in which molecular momentum of the metal nanomaterials 2a included in the matrix resin of the prepreg 2 is increased by applying a magnetic field MF to the prepreg 2 (resin composite material) using the magnetic field applying coil 33. The material of the metal nanomaterial 2a is required so as to be self-heated by applying the magnetic field MF. The magnetic field applying coil 33 forms a magnetic field MF by the current which is supplied from the power source 35 via the power lines 36.

Similarly to the pressurizing bodies 14 of the curing device 11 in the second embodiment, the plurality of pressurizing bodies 34 are continuously provided in the predetermined length (for example, the longitudinal direction) of the prepreg 2. The material of each of the pressurizing bodies 34 is required to be a material which is not easily self-heated, that is, is not heated to a temperature at which a function as the pressurizing body is damaged even when placed in an environment to which the magnetic field MF is applied. Each of the plurality of pressurizing bodies 34 can come into pressure-contact with and can be separated from the surface of the prepreg 2 individually by an actuator (not shown).

A curing method for the prepreg 2 is performed as follows by the curing device 31 configured as above. This curing method is also performed by a procedure according to the flowchart shown in Fig. 3.

First, the uncured prepreg 2 is laminated (placed, wound, or the like) on the forming base 32, a predetermined molding tool, a jig, or the like (lamination step S1).

Next, the magnetic field applying coil 33 applies the magnetic field MF to the prepreg 2 (heating step S2). If the magnetic field MF is applied to the prepreg 2, the molecular momentum of the metal nanomaterials 2a added to the matrix resin of the prepreg 2 increases, and the metal nanomaterials 2a are self-heated. This heat is transmitted to the matrix resin of the prepreg 2, and the matrix resin is softened or melted.

Meanwhile, since each of the pressurizing bodies 34 is formed of a material which is not easily self-heated even when it is applied by the magnetic fields MF, the temperature of the pressurizing body 34 is not increased unlike the prepreg 2. Accordingly, a function as the pressurizing body is not damaged.

Next, the pressurizing bodies 34 come into pressure-contact with the surface of the uncured prepreg 2 by driving the actuators (not shown) to pressurize the surface (pressurization step S3). In this case, a pressurizing force is set such that bubbles included inside the prepreg 2 are extracted to the outside.

Bubbles of air included inside the prepreg 2 or bubbles of gas generated during heating are extracted from the center portion of the prepreg 2 toward the peripheral portion thereof by the pressurization of the pressurizing bodies 34 so as to be removed.

In a case where the matrix resin of the prepreg 2 is a thermosetting resin, a curing reaction is generated by an increase in the temperature due to the self-heating of the metal nanomaterials 2a and the pressurization of the pressurizing body 6, and the matrix resin is cured by the curing reaction. Meanwhile, in a case where the matrix resin of the prepreg 2 is a thermoplastic resin, the matrix resin is cooled after being heated and is cured. If the pressurization is completed, the actuators are driven to lift the pressurizing bodies 34.

For example, in a case where the prepreg 2 is a sheet shape or an elongated shape, one pressurizing body 34 comes into pressure-contact with one location (for example, center portion) of the prepreg 2, and other pressurizing bodies 34 adjacent to the one pressurizing body 34 sequentially come into pressure-contact with the prepreg 2. Accordingly, a curing range of the prepreg 2 is widened, bubbles of air or gas included in the prepreg 2 are extracted to the end portion sides of the prepreg 2, and favorable degassing can be achieved.

In this way, by alternately performing the heating (heating step S2) by applying the magnetic field MF and the pressurization (pressurization step S3) by the pressurizing bodies 34 a plurality of times and by sequentially pressurizing the plurality of pressurizing bodies 34 from the center portion toward the outside in the pressurization step S3, it is possible to form the molded resin article such that the entire prepreg 2 is uniformly cured. Applying the magnetic field MF and the pressurization of the pressurizing bodies 34 may be simultaneously performed.

Finally, the molded resin article is separated from the forming base 32 and the molded resin article is completed (mold release step S4).

Compared to the cases where the electromagnetic waves are emitted or the electric fields are applied like the above-described embodiments, in the curing device 31 having the above-described configuration, effects of self-heating the metal nanomaterials 2a include in the prepreg 2 are inferior. However, the configuration of the curing equipment can be simpler and cheaper. Accordingly, it is possible to effectively heat and cure the prepreg 2 according to properties of the used metal nanomaterials 2a.

As described above, in the curing devices 1, 11, 21, and 31 for a resin composite material and the curing methods according to the present invention, the resin composite material is cured by applying a specific environment (electromagnetic waves EW, electric field EF, magnetic field MF, or the like) in which the molecular momentum of an object increases to the resin composite material such as the prepreg 2 and by self-heating the metal nanomaterials 2a included in the resin composite material.

According to the curing devices 1, 11, 21, and 31 and the curing methods, since evacuation using a vacuum bag or heating using a heater such as an autoclave is not required, it is possible to save labor when the resin composite material is molded, simplify the curing equipment, save energy, and decrease manufacturing costs of the molded resin article.

The present invention is not limited to the embodiments; modifications and improvements can be appropriately applied within the scope of the claims.

For example, in the above-described embodiments, the cases where the prepreg in which the matrix resin is half-cured is cured and molded are described. However, the present invention can be applied to other resin composite materials. The configurations of the above-described embodiments may be combined.

In addition, in the above-described embodiments, each of the pressurizing bodies 6, 14, 28, and 34 is a surface-pressure type (stamp type) pressurizing body. However, the pressurizing body may be changed from the surface-pressure type pressurizing body to a rolling type (roller type) pressurizing body.

In addition, the environment setting units of the above-described embodiments may be combined. For example, the electromagnetic wave irradiation unit of the first embodiment, the electrode of the second embodiment, and the magnetic field applying coil of the third embodiment may be used so as to be appropriately combined.

### Reference Signs List

1, 11, 21, 31: curing device
2: prepreg (resin composite material)
2a: metal nanomaterial
5: electromagnetic wave irradiation unit (environment setting unit)
6, 14, 28, 34: pressurizing body
7: actuator (pressure-contact driving unit)
13A, 13B, 23A, 23B, 24A, 24B, 25A, 25B, 26A, 26B, 27A, 27B: electrode (environment setting unit)
33: magnetic field applying coil (environment setting unit)
EW: electromagnetic wave
EF: electric field
MF: magnetic field
S2: heating step
S3: pressurization step

## Claims

1. A curing device (1) for a resin composite material (2), the curing device (1) comprising:
an environment setting unit (5) which is adapted for applying a specific physical environment to an uncured resin composite material (2) including a fibrous reinforcing material, a thermosetting or thermoplastic matrix resin, and a metal nanomaterial (2a) selected from platinum, gold, nickel and copper, wherein the specific physical environment is such that the molecular momentum of the metal nanomaterial (2a) is increased and the metal nanomaterial (2a) self-heats when placed in the specific physical environment;
a pressurizing body (6) which is adapted for coming into pressure-contact with a surface of the resin composite material (2); and
a pressure-contact driving unit (7) which allows the pressurizing body (6) to come into pressure-contact with the surface of the resin composite material (2),
wherein the environment setting unit (5) is an electromagnetic wave irradiation unit, and the specific physical environment is a state where electromagnetic waves (EW) are emitted by the electromagnetic wave irradiation unit.

2. A curing device (11, 21) for a resin composite material (2), the curing device (11, 21) comprising:
an environment setting unit (13A, 13B, 23A, 23B, 24A, 24B, 25A, 25B, 26A, 26B, 27A, 27B) which is adapted for applying a specific physical environment to an uncured resin composite material (2) including a fibrous reinforcing material, a thermosetting or thermoplastic matrix resin, and a metal nanomaterial (2a) selected from platinum, gold, nickel and copper, wherein the specific physical environment is such that the molecular momentum of the metal nanomaterial (2a) is increased and the metal nanomaterial (2a) self-heats when placed in the specific physical environment;
a pressurizing body (14, 28) which is adapted for coming into pressure-contact with a surface of the resin composite material (2); and
a pressure-contact driving unit (7) which allows the pressurizing body (14, 28) to come into pressure-contact with the surface of the resin composite material (2),
wherein the environment setting unit (13A, 13B, 23A, 23B, 24A, 24B, 25A, 25B, 26A, 26B, 27A, 27B) is a plurality of electrodes which directly face each other in a state where the resin composite material (2) is interposed between the electrodes, and the specific physical environment is a state where an electric field (EF) is applied to portions between the plurality of electrodes.

3. A curing device (31) for a resin composite material (2), the curing device (31) comprising:
an environment setting unit (33) which is adapted for applying a specific physical environment to an uncured resin composite material (2) including a fibrous reinforcing material, a thermosetting or thermoplastic matrix resin, and a metal nanomaterial (2a) selected from platinum, gold, nickel and copper, wherein the specific physical environment is such that the molecular momentum of the metal nanomaterial (2a) is increased and the metal nanomaterial (2a) self-heats when placed in the specific physical environment;
a pressurizing body (34) which is adapted for coming into pressure-contact with a surface of the resin composite material (2); and
a pressure-contact driving unit (7) which allows the pressurizing body (34) to come into pressure-contact with the surface of the resin composite material (2),
wherein the environment setting unit (33) is a magnetic field applying coil, and the specific physical environment is a state where a magnetic field (MF) is applied to the resin composite material (2) by the magnetic field applying coil.

4. The curing device (1, 11, 21, 31) for a resin composite material (2) according to any one of claims 1 to 3, wherein a material of the pressurizing body (6, 14, 28, 34) is any one of quartz glass, a polymer material, and a ceramics material.

5. The curing device (1, 11, 21, 31) for a resin composite material (2) according to any one of claims 1 to 4, wherein a plurality of pressurizing bodies (6, 14, 28, 34) are continuously provided in a predetermined direction of the resin composite material (2) and a pressurizing body (6, 14, 28, 34) positioned at an arbitrary position among the pressurizing bodies (6, 14, 28, 34) can be operated.

6. The curing device (1, 11, 21, 31) for a resin composite material (2) according to any one of claims 1 to 5, wherein a plurality of environment setting units (5, 13A, 13B, 23A, 23B, 24A, 24B, 25A, 25B, 26A, 26B, 27A, 27B, 33) are continuously provided in a predetermined direction of the resin composite material (2) and an environment setting unit (5, 13A, 13B, 23A, 23B, 24A, 24B, 25A, 25B, 26A, 26B, 27A, 27B, 33) positioned at an arbitrary position among the environment setting units (5, 13A, 13B, 23A, 23B, 24A, 24B, 25A, 25B, 26A, 26B, 27A, 27B, 33) can be operated.

7. A curing method for a resin composite material (2), the curing method comprising:
a heating step (S2) of applying a specific physical environment to an uncured resin composite material (2) including a fibrous reinforcing material, a thermosetting or thermoplastic matrix resin, and a metal nanomaterial (2a) selected from platinum, gold, nickel and copper, wherein the specific physical environment is such that the molecular momentum of the metal nanomaterial (2a) is increased and the metal nanomaterial (2a) self-heats, and heating the resin composite material (2); and
a pressurization step (S3) of allowing a pressurizing body (6) to come into pressure-contact with the surface of the heated resin composite material (2) and pressurizing the heated resin composite material (2),
wherein the specific physical environment is a state where electromagnetic waves (EW) are emitted.

8. A curing method for a resin composite material (2), the curing method comprising:
a heating step (S2) of applying a specific physical environment to an uncured resin composite material (2) including a fibrous reinforcing material, a thermosetting or thermoplastic matrix resin, and a metal nanomaterial (2a) selected from platinum, gold, nickel and copper, wherein the specific physical environment is such that the molecular momentum of the metal nanomaterial (2a) is increased and the metal nanomaterial (2a) self-heats, and heating the resin composite material (2); and
a pressurization step (S3) of allowing a pressurizing body (14, 28) to come into pressure-contact with the surface of the heated resin composite material (2) and pressurizing the heated resin composite material (2),
wherein the specific physical environment is a state where an electric field (EF) is applied.

9. A curing method for a resin composite material (2), the curing method comprising:
a heating step (S2) of applying a specific physical environment to an uncured resin composite material (2) including a fibrous reinforcing material, a thermosetting or thermoplastic matrix resin, and a metal nanomaterial (2a) selected from platinum, gold, nickel and copper, wherein the specific physical environment is such that the molecular momentum of the metal nanomaterial (2a) is increased and the metal nanomaterial (2a) self-heats, and heating the resin composite material (2); and
a pressurization step (S3) of allowing a pressurizing body (34) to come into pressure-contact with the surface of the heated resin composite material (2) and pressurizing the heated resin composite material (2),
wherein the specific physical environment is a state where a magnetic field (MF) is applied.

10. The curing method for a resin composite material (2) according to any one of claims 7 to 9, wherein the heating step (S2) and the pressurization step (S3) are alternately performed a plurality of times.

## Patentansprüche

1. Eine Aushärtevorrichtung (1) für ein Harzverbundmaterial (2), wobei die Aushärtevorrichtung (1) umfasst:
eine Umgebungseinstelleinheit (5), die dazu angepasst ist, eine spezifische physikalische Umgebung auf ein ungehärtetes Harzverbundmaterial (2) anzuwenden, das ein faseriges Verstärkungsmaterial, ein duroplastisches oder thermoplastisches Matrixharz und ein metallisches Nanomaterial (2a), ausgewählt aus Platin, Gold, Nickel und Kupfer enthält, wobei die spezifische physikalische Umgebung so ist, dass das Molekular-Moment des metallischen Nanomaterials (2a) erhöht wird und sich das metallische Nanomaterial (2a) selbst erwärmt, wenn es in die spezifische physikalische Umgebung gebracht wird;
einen Druckkörper (6), der ausgestaltet ist, um in Druckkontakt mit einer Oberfläche des Harzverbundmaterials (2) zu kommen; und
eine Druckkontakt-Antriebseinheit (7), die es dem Druckkörper (6) ermöglicht, in Druckkontakt mit der Oberfläche des Harzverbundmaterials (2) zu kommen,
wobei die Umgebungseinstelleinheit (5) eine Bestrahlungseinheit mit elektromagnetischen Wellen ist und die spezifische physikalische Umgebung ein Zustand ist, in dem elektromagnetische Wellen (EW) von der Bestrahlungseinheit für elektromagnetische Wellen emittiert werden.

2. Eine Aushärtevorrichtung (11, 21) für ein Harzverbundmaterial (2), wobei die Aushärtevorrichtung (11, 21) umfasst:
eine Umgebungseinstelleinheit ( 13A, 13B, 23A, 23B, 24A, 24B, 25A, 25B, 26A, 26B, 27A, 27B), die dazu angepasst ist, eine spezifische physikalische Umgebung auf ein ungehärtetes Harzverbundmaterial (2), das ein faseriges Verstärkungsmaterial, ein duroplastisches oder thermoplastisches Matrixharz und ein metallisches Nanomaterial (2a), ausgewählt aus Platin, Gold, Nickel und Kupfer enthält, wobei die spezifische physikalische Umgebung so ist, dass das Molekular-Moment des metallischen Nanomaterials (2a) erhöht wird und sich das metallische Nanomaterial (2a) selbst erwärmt, wenn es in die spezifische physikalische Umgebung gebracht wird;
einen Druckkörper (14, 28), der ausgestaltet ist, um in Druckkontakt mit einer Oberfläche des Harzverbundmaterials (2) zu kommen; und
eine Druckkontakt-Antriebseinheit (7), die es dem Druckkörper (14, 28) ermöglicht, in Druckkontakt mit der Oberfläche des Harzverbundmaterials (2) zu kommen,
wobei die Umgebungseinstelleinheit (13A, 13B, 23A, 23B, 24A, 24B, 25A, 25B, 26A, 26B, 27A, 27B) aus mehreren Elektroden besteht, die einander in einem Zustand direkt gegenüberstehen, in dem das Harzverbundmaterial (2) zwischen den Elektroden angeordnet ist, und wobei die spezifische physikalische Umgebung ein Zustand ist, in dem ein elektrisches Feld (EF) an Abschnitte zwischen den mehreren Elektroden angelegt wird.

3. Eine Aushärtevorrichtung (31) für ein Harzverbundmaterial (2), wobei die Aushärtevorrichtung (31) umfasst:
eine Umgebungseinstelleinheit (5), die dazu angepasst ist, eine spezifische physikalische Umgebung auf ein ungehärtetes Harzverbundmaterial (2) anzuwenden, das ein faseriges Verstärkungsmaterial, ein duroplastisches oder thermoplastisches Matrixharz und ein metallisches Nanomaterial (2a), ausgewählt aus Platin, Gold, Nickel und Kupfer enthält, wobei die spezifische physikalische Umgebung so ist, dass das Molekular-Moment des metallischen Nanomaterials (2a) erhöht wird und sich das metallische Nanomaterial (2a) selbst erwärmt, wenn es in die spezifische physikalische Umgebung gebracht wird;
einen Druckkörper (6), der ausgestaltet ist, um in Druckkontakt mit einer Oberfläche des Harzverbundmaterials (2) zu kommen; und
eine Druckkontakt-Antriebseinheit (7), die es dem Druckkörper (6) ermöglicht, in Druckkontakt mit der Oberfläche des Harzverbundmaterials (2) zu kommen,
wobei die Umgebungseinstelleinheit (33) eine Spule zum Anlegen eines Magnetfelds ist und die spezifische physikalische Umgebung ein Zustand ist, in dem durch die Spule zum Anlegen eines Magnetfelds ein Magnetfeld (MF) an das Harzverbundmaterial (2) angelegt wird.

4. Die Aushärtevorrichtung (1, 11, 21, 31) für ein Harzverbundmaterial (2) nach einem der Ansprüche 1 bis 3, wobei ein Material des Druckkörpers (6, 14, 28, 34) ein Quarzglas, ein Polymermaterial oder ein Keramikmaterial ist.

5. Die Aushärtevorrichtung (1, 11, 21, 31) für ein Harzverbundmaterial (2) nach einem der Ansprüche 1 bis 4, wobei mehrere Druckkörper (6, 14, 28, 34) kontinuierlich in einer vorbestimmten Richtung des Harzverbundmaterials (2) vorgesehen sind, und wobei ein Druckkörper (6, 14, 28, 34), der an einer beliebigen Position unter den Druckkörpern (6, 14, 28, 34) positioniert ist, betätigt werden kann.

6. Die Aushärtevorrichtung (1, 11, 21, 31) für ein Harzverbundmaterial (2) nach einem der Ansprüche 1 bis 5, wobei eine Mehrzahl von Umgebungseinstelleinheiten (5, 13A, 13B, 23A, 23B, 24A, 24B, 25A, 25B, 26A, 26B, 27A, 27B, 33) kontinuierlich in einer vorbestimmten Richtung des Harzverbundmaterials (2) vorgesehen sind, und wobei eine Umgebungseinstelleinheit (5, 13A, 13B, 23A, 23B, 24A, 24B, 25A, 25B, 26A, 26B, 27A, 27B, 33), die an einer beliebigen Position unter den Umgebungseinstelleinheiten (5, 13A, 13B, 23A, 23B, 24A, 24B, 25A, 25B, 26A, 26B, 27A, 27B, 33) positioniert ist, betätigt werden können.

7. Ein Aushärtungsverfahren für ein Harzverbundmaterial (2), wobei das Aushärtungsverfahren Folgendes umfasst:
einen Erwärmungsschritt (S2) zum Anwenden einer spezifischen physikalischen Umgebung auf ein ungehärtetes Harzverbundmaterial (2), das ein faseriges Verstärkungsmaterial, ein duroplastisches oder thermoplastisches Matrixharz und ein metallisches Nanomaterial (2a), ausgewählt aus Platin, Gold, Nickel und Kupfer enthält, wobei die spezifische physikalische Umgebung so ist, dass das Molekular-Moment des metallischen Nanomaterials (2a) erhöht wird und sich das metallische Nanomaterial (2a) selbst erwärmt, wenn es in die spezifische physikalische Umgebung gebracht wird; Und
einen Druckanwendungsschritt (S3), bei dem es einem Druckkörper (6) ermöglicht wird, in Druckkontakt mit der Oberfläche des erhitzten Harzverbundmaterials (2) zu kommen, und das erhitzte Harzverbundmaterial (2) unter Druck gesetzt wird,
wobei die spezifische physikalische Umgebung ein Zustand ist, in dem elektromagnetische Wellen (EW) emittiert werden.

8. Ein Aushärtungsverfahren für ein Harzverbundmaterial (2), wobei das Aushärtungsverfahren Folgendes umfasst:
einen Erwärmungsschritt (S2) zum Anwenden einer spezifischen physikalischen Umgebung auf ein ungehärtetes Harzverbundmaterial (2), das ein faseriges Verstärkungsmaterial, ein duroplastisches oder thermoplastisches Matrixharz und ein metallisches Nanomaterial (2a), ausgewählt aus Platin, Gold, Nickel und Kupfer enthält, wobei die spezifische physikalische Umgebung so ist, dass das Molekular-Moment des metallischen Nanomaterials (2a) erhöht wird und sich das metallische Nanomaterial (2a) selbst erwärmt, wenn es in die spezifische physikalische Umgebung gebracht wird; Und
einen Druckanwendungsschritt (S3), bei dem es einem Druckkörper (6) ermöglicht wird, in Druckkontakt mit der Oberfläche des erhitzten Harzverbundmaterials (2) zu kommen, und das erhitzte Harzverbundmaterial (2) unter Druck gesetzt wird,
wobei die spezifische physikalische Umgebung ein Zustand ist, in dem ein elektrisches Feld (EF) angelegt wird.

9. Ein Aushärtungsverfahren für ein Harzverbundmaterial (2), wobei das Aushärtungsverfahren Folgendes umfasst:
einen Erwärmungsschritt (S2) zum Anwenden einer spezifischen physikalischen Umgebung auf ein ungehärtetes Harzverbundmaterial (2), das ein faseriges Verstärkungsmaterial, ein duroplastisches oder thermoplastisches Matrixharz und ein metallisches Nanomaterial (2a), ausgewählt aus Platin, Gold, Nickel und Kupfer enthält, wobei die spezifische physikalische Umgebung so ist, dass das Molekular-Moment des metallischen Nanomaterials (2a) erhöht wird und sich das metallische Nanomaterial (2a) selbst erwärmt, wenn es in die spezifische physikalische Umgebung gebracht wird; Und
einen Druckanwendungsschritt (S3), bei dem es einem Druckkörper (6) ermöglicht wird, in Druckkontakt mit der Oberfläche des erhitzten Harzverbundmaterials (2) zu kommen, und das erhitzte Harzverbundmaterial (2) unter Druck gesetzt wird,
wobei die spezifische physikalische Umgebung ein Zustand ist, in dem ein Magnetfeld (MF) angelegt wird.

10. Das Aushärtungsverfahren für ein Harzverbundmaterial (2) nach einem der Ansprüche 7 bis 9, wobei der Erwärmungsschritt (S2) und der Druckbeaufschlagungsschritt (S3) mehrmals abwechselnd durchgeführt werden.

## Revendications

1. Dispositif de durcissement (1) pour un matériau composite en résine (2), le dispositif de durcissement (1) comprenant :
une unité de réglage d'environnement (5) qui est adaptée pour appliquer un environnement physique spécifique à un matériau composite en résine non durci (2) comportant un matériau de renforcement fibreux, une résine matricielle thermodurcissable ou thermoplastique, et un nanomatériau métallique (2a) choisi parmi le platine, l'or, le nickel et le cuivre, dans lequel l'environnement physique spécifique est tel que l'impulsion moléculaire du nanomatériau métallique (2a) augmente et le nanomatériau métallique (2a) s'autochauffe lorsqu'il est placé dans l'environnement physique spécifique ;
un corps de mise sous pression (6) qui est adapté pour entrer en contact par pression avec une surface du matériau composite en résine (2) ; et
une unité d'entraînement par contact par pression (7) qui permet au corps de mise sous pression (6) d'entrer en contact par pression avec la surface du matériau composite en résine (2),
dans lequel l'unité de réglage d'environnement (5) est une unité d'irradiation par ondes électromagnétiques, et l'environnement physique spécifique est un état dans lequel des ondes électromagnétiques (EW) sont émises par l'unité d'irradiation par ondes électromagnétiques.

2. Dispositif de durcissement (11, 21) pour un matériau composite en résine (2), le dispositif de durcissement (11, 21) comprenant :
une unité de réglage d'environnement (13A, 13B, 23A, 23B, 24A, 24B, 25A, 25B, 26A, 26B, 27A, 27B) qui est adapté pour appliquer un environnement physique spécifique à un matériau composite en résine non durci (2) comportant un matériau de renforcement fibreux, une résine matricielle thermodurcissable ou thermoplastique, et un nanomatériau métallique (2a) choisi parmi le platine, l'or, le nickel et le cuivre, dans lequel l'environnement physique spécifique est tel que l'impulsion moléculaire du nanomatériau métallique (2a) augmente et que le nanomatériau métallique (2a) s'autochauffe lorsqu'il est placé dans l'environnement physique spécifique ;
un corps de mise sous pression (14, 28) qui est adapté pour entrer en contact par pression avec une surface du matériau composite en résine (2) ; et
une unité d'entraînement par contact par pression (7) qui permet au corps de mise sous pression (14, 28) d'entrer en contact par pression avec la surface du matériau composite en résine (2),
dans lequel l'unité de réglage de l'environnement (13A, 13B, 23A, 23B, 24A, 24B, 25A, 25B, 26A, 26B, 27A, 27B) est une pluralité d'électrodes qui se font directement face dans un état dans lequel le matériau composite en résine (2) est interposé entre les électrodes, et l'environnement physique spécifique est un état dans lequel un champ électrique (EF) est appliqué à des parties situées entre la pluralité d'électrodes.

3. Dispositif de durcissement (31) pour un matériau composite en résine (2), le dispositif de durcissement (31) comprenant :
une unité de réglage d'environnement (33) qui est adaptée pour appliquer un environnement physique spécifique à un matériau composite en résine non durci (2) comportant un matériau de renforcement fibreux, une résine matricielle thermodurcissable ou thermoplastique, et un nanomatériau métallique (2a) choisi parmi le platine, l'or, le nickel et le cuivre, dans lequel l'environnement physique spécifique est tel que l'impulsion moléculaire du nanomatériau métallique (2a) augmente et le nanomatériau métallique (2a) s'autochauffe lorsqu'il est placé dans l'environnement physique spécifique ;
un corps de mise sous pression (34) qui est adapté pour entrer en contact par pression avec une surface du matériau composite en résine (2) ; et
une unité d'entraînement par contact par pression (7) qui permet au corps de mise sous pression (34) d'entrer en contact par pression avec la surface du matériau composite en résine (2),
dans lequel l'unité de réglage d'environnement (33) est une bobine appliquant un champ magnétique, et l'environnement physique spécifique est un état dans lequel un champ magnétique (MF) est appliqué au matériau composite en résine (2) par la bobine appliquant un champ magnétique.

4. Dispositif de durcissement (1, 11, 21, 31) pour un matériau composite en résine (2) selon l'une quelconque des revendications 1 à 3, dans lequel un matériau du corps de mise sous pression (6, 14, 28, 34) est l'un quelconque parmi un verre de quartz, un matériau polymère et un matériau céramique.

5. Dispositif de durcissement (1, 11, 21, 31) pour un matériau composite en résine (2) selon l'une quelconque des revendications 1 à 4, dans lequel une pluralité de corps de mise sous pression (6, 14, 28, 34) sont prévus en continu dans une direction prédéterminée du matériau composite en résine (2) et un corps de mise sous pression (6, 14, 28, 34) positionné à une position arbitraire parmi les corps de mise sous pression (6, 14, 28, 34) peut être actionné.

6. Dispositif de durcissement (1, 11, 21, 31) pour un matériau composite en résine (2) selon l'une quelconque des revendications 1 à 5, dans lequel une pluralité d'unités de réglage de l'environnement (5, 13A, 13B, 23A, 23B, 24A, 24B, 25A, 25B, 26A, 26B, 27A, 27B, 33) sont disposées en continu dans une direction prédéterminée du matériau composite en résine (2) et une unité de réglage d'environnement (5, 13A, 13B, 23A, 23B, 24A, 24B, 25A, 25B, 26A, 26B, 27A, 27B, 33) positionnée à une position arbitraire parmi les unités de réglage d'environnement (5, 13A, 13B, 23A, 23B, 24A, 24B, 25A, 25B, 26A, 26B, 27A, 27B, 33) peuvent être actionnés.

7. Procédé de durcissement pour un matériau composite à base résine (2), le procédé de durcissement comprenant :
une étape de chauffage (S2) consistant à appliquer un environnement physique spécifique à un matériau composite en résine non durci (2) comportant un matériau de renforcement fibreux, un matériau thermodurcissable ou thermoplastique, une résine matricielle et un nanomatériau métallique (2a) choisi parmi le platine, l'or, le nickel et le cuivre, dans lequel l'environnement physique spécifique est tel que l'impulsion moléculaire du nanomatériau métallique (2a) augmente et que le nanomatériau métallique (2a) s'autochauffe, et chauffer le matériau composite en résine (2) ; et
une étape de mise sous pression (S3) pour permettre à un corps de mise sous pression (6) d'entrer en contact par pression avec la surface du matériau composite en résine chauffé (2) et de mettre sous pression le matériau composite en résine chauffé (2),
dans lequel l'environnement physique spécifique est un état dans lequel des ondes électromagnétiques (EW) sont émises.

8. Procédé de durcissement pour un matériau composite en résine (2), le procédé de durcissement comprenant :
une étape de chauffage (S2) pour appliquer un environnement physique spécifique à un matériau composite en résine non durci (2) comportant un matériau de renforcement fibreux, une résine matricielle thermodurcissable ou thermoplastique, et un nanomatériau métallique (2a) choisi parmi le platine, l'or, le nickel et le cuivre, dans lequel l'environnement physique spécifique est tel que l'impulsion moléculaire du nanomatériau métallique (2a) augmente et que le nanomatériau métallique (2a) s'autochauffe, et chauffer le matériau composite en résine (2) ; et
une étape de mise sous pression (S3) pour permettre à un corps de mise sous pression (14, 28) d'entrer en contact par pression avec la surface du matériau composite en résine chauffé (2) et de mettre sous pression le matériau composite en résine chauffé (2),
dans lequel l'environnement physique spécifique est un état dans lequel un champ électrique (EF) est appliqué.

9. Procédé de durcissement pour un matériau composite en résine (2), le procédé de durcissement comprenant :
une étape de chauffage (S2) pour appliquer un environnement physique spécifique à un matériau composite en résine non durci (2) comportant un matériau de renforcement fibreux, une résine matricielle thermodurcissable ou thermoplastique, et un nanomatériau métallique (2a) choisi parmi le platine, l'or, le nickel et le cuivre, dans lequel l'environnement physique spécifique est tel que l'impulsion moléculaire du nanomatériau métallique (2a) augmente et que le nanomatériau métallique (2a) s'autochauffe, et chauffer le matériau composite en résine (2) ; et
une étape de mise sous pression (S3) pour permettre à un corps de mise sous pression (34) d'entrer en contact par pression avec la surface du matériau composite en résine chauffé (2) et de mettre sous pression le matériau composite en résine chauffé (2),
dans lequel l'environnement physique spécifique est un état dans lequel un champ magnétique (MF) est appliqué.

10. Procédé de durcissement d'un matériau composite en résine (2) selon l'une quelconque des revendications 7 à 9, dans lequel l'étape de chauffage (S2) et l'étape de mise sous pression (S3) sont réalisées en alternance plusieurs fois.
